# EUROPEAN PATENT APPLICATION

(11) **EP 1 864 620 A2**
(43) Date of publication of application: **12.12.2007**
(21) Application number: 07252208.9
(22) Date of filing: 30.05.2007
(51) Int. Cl.: A61B 17/70

(54) **A fixation device for an elongate spinal support element**

(30) Priority: 06.06.2006 GB 0611073; 22.03.2007 GB 0705463
(71) Applicant: Depuy International Limited, Beeston, Leeds LS11 8DT (GB)
(72) Inventor: Sanders, Marc, 7553 AM Hengelo (NL)
(74) Representative: Belcher, Simon James

(57) **Abstract**

A fixation device for connecting an elongate support element to a patient's vertebra in order to correct a spinal deformity includes a housing for the support element formed by a channel in which the element can be received. A transverse arm having a housing end and a free end is connected to a side wall of the channel housing at its housing end. The transverse arm has a first recess formed in it towards its free end, and the housing or the transverse arm at or towards the housing end has a second recess formed in it. The device includes a first wire which is a close fit in the first recess and can engage a vertebra so that the transverse arm is connected directly to the vertebra towards its free end, and a second wire which is a close fit in the second recess and can engage a vertebra so that the device is connected directly to the vertebra at the housing at or towards the housing end of the transverse arm.

## Description

This invention relates to a fixation device for connecting an elongate support element to a patient's vertebra, and to an implant kit which includes a fixation device.

Support elements can be used to correct serious shape deformations of the spinal column and chest of a patient. Examples of serious shape deformations of the spinal column include lateral curvature and axial turning.

EP-A-470660 discloses apparatus for correcting the shape of a spinal column. The apparatus includes a rod having a square cross-section. The rod is formed from a nickel-titanium alloy which has been treated so that it exhibits shape memory properties, in bending or in torsion or both. Articles formed from shape memory alloys can exhibit shape memory properties associated with transformations between martensite and austenite phases of the alloys. These properties include thermally induced changes in configuration in which an article is first deformed from a heat-stable configuration to a heat-unstable configuration while the alloy is in its martensite phase. Subsequent exposure to increased temperature results in a change in configuration from the heat-unstable configuration towards the original heat-stable configuration as the alloy reverts from its martensite phase to its austenite phase. The transformation from austenite to martensite on cooling begins at a temperature known as the Mₛ temperature, and is completed at a temperature known as the M_{f} temperature. The transformation of martensite to austenite upon heating begins at a temperature known as the Aₛ temperature and is complete at a temperature known as the A_{f} temperature.

The rod of the apparatus disclosed in EP-A-470660 is fastened to a patient's vertebrae while in the configuration from which it has to recover. The rod can be bent and twisted so as to correspond to the curve or torsion of the deformed spinal column. The temperature of the rod is then increased so that it is greater than the Aₛ temperature of the alloy. The rod then recovers towards its heat-stable configuration, applying a corrective force to the spinal column.

Rods or other support elements which are used to support a spinal column can be fastened to the patient's vertebrae by means of fastening devices such as screws or hooks. Such devices can include a channel in which the support element can be fitted, and retained by means of a lock screw. The channel can have a through hole in its base, through which the shank of a fixation screw can extend. The channel can have a hook which extends beyond the base of the channel, to engage a projection provided by the bone tissue of the vertebra.

The axial rotation of a spinal column can be corrected by fastening a support element to the vertebrae in a configuration which corresponds to the shape of a deformed spinal column. In order to correct axial rotation of the spinal column, the fixation device must be able to be fastened to the vertebrae so as to prevent rotation of the rod relative to the vertebrae. As a result, the fixation device can be subjected to significant corrective forces.

It is important to ensure that the support element (for example, a rod) is fastened securely to the vertebrae, and that the tissue to which the rod is fastened is capable of carrying the imposed corrective forces. Problems that can arise due to inappropriate fixation include pull-out of a fixation screw, fracture of a fixation screw, and fracture of a vertebra into which the screw is fixed.

The load experienced by a fixation device can be reduced by securing two rods positioned on either side of the spinal column. Each of the rods is secured to the vertebrae by separate fixation devices. Although this apparatus can be used to correct the lateral deformation of a spinal column, it is unable to correct the axial rotation of the spine.

It can be preferred to spread the load that is applied by a rod between two fastening devices. This can be done using a bridge connector which includes a channel in which the support element can be fitted, and a pair of transverse arms. Each of the transverse arms can be fastened to a vertebra by means of two bone screws or other fastening devices. This arrangement has been found to be satisfactory to secure a support rod to vertebrae in the sacral, lumbar, and lower thoracic regions of the spinal column. However, the use of this fastening arrangement in the upper thoracic and cervical region can be difficult because of the small size of the vertebrae.

When the spinal column is severely deformed, for example severely curved it is difficult to secure the rod in position using pedicle screws or hooks due to a number of factors including the lack of available space surrounding the vertebrae and the degree of curvature of the spine. Wires can be used to secure the rods to the vertebrae in situations where there is insufficient space available to use pedicle screws or hooks. Wires are however unable to prevent the rod (or other support element) from rotating relative to the vertebrae. Wires are therefore unable to provide good torsional stability of the rod (or other support element) and there is a risk that the rod will be able to rotate relative to the vertebrae.

The present invention provides a fixation device for connecting an elongate support element to a patient's vertebra in order to correct a spinal deformity, including lateral deformation and axial rotation of the spine, which includes a housing for the support element, especially a rod, formed by a channel, and a transverse arm connected to a side wall of the channel housing, in which the transverse arm provides a recess for receiving a wire which can engage a vertebra so that the transverse arm is connected directly to the vertebra.

Accordingly, in one aspect, the invention provides a fixation device for connecting an elongate support element to a patient's vertebra in order to correct a spinal deformity, which comprises:
a. a housing for the support element formed by a channel in which the element can be received,
b. a transverse arm having a housing end and a free end, which is connected to a side wall of the channel housing at its housing end, in which the transverse arm has a first recess formed in it towards its free end, and in which the housing or the transverse arm at or towards the housing end has a second recess formed in it,
c. a first wire which is a close fit in the first recess and can engage a vertebra so that the transverse arm is connected directly to the vertebra towards its free end,
d. a second wire which is a close fit in the second recess and can engage a vertebra so that the device is connected directly to the vertebra at the housing at or towards the housing end of the transverse arm.

The fixation device of the invention has the advantage that the support element can be secured to a vertebra with low risk of the support element being able to rotate relative to the spinal column. The fixation device therefore provides good torsional stability of the support element relative to the spinal column. The fixation device can be used to transfer the desired torsional forces to correct the axial rotation of the spinal column from the support element to a vertebra. The fixation device therefore provides improved correction of axial rotation of a spinal column.

The fixation device also has the advantage that the surgeon can adjust the correctional torsional forces which are transferred to a vertebra from the support element by loosening or tightening the wire as required.

Furthermore, the invention has the advantage that the fixation device can be secured to a vertebra in circumstances where fixation elements such as fixation screws and hooks cannot be used. For example, the fixation device can be attached to a vertebra using wire in circumstances where there is limited available space around the vertebrae or where it is difficult to attach the support element due to the severe curvature of the spine or where the bone tissue is weakened such that a screw fastener cannot be used safely.

As the wire can be looped around the vertebra, the fixation device also has a reduced risk of becoming loose from the vertebrae as a result of pull-out of the fixation device from the vertebra. Furthermore, the invention also has the advantage that the forces exerted by the fixation device are spread out over a large area of the vertebra as a result of the wire being looped around the vertebra. There is therefore a reduced risk of the bone adjacent to the fixation device fracturing as a result of the wire being looped around the vertebra.

In another aspect, the invention provides an implant kit which includes a fixation device as discussed above and an elongate support element.

The channel formed by the housing can have at least one flat internal face in order to reduce the risk of rotation of the support element within the channel. The flat internal face can be an internal face or at least a portion of an internal face which is generally flat or less rounded than the other internal faces of the channel. The channel can have a cross-section which is polygonal in shape when viewed along a line joining the two ends of the channel. For example, the polygonal shape can have at least four faces, including square or rectangular, or trapezoidal, or with six or eight or more faces.

The elongate support element can be secured within the channel of the housing by a fixation element which engages the support element. Examples of suitable fixation elements include a screw extending through a surface of the channel housing or a wire looped around the channel housing. The fixation element secures the support element within position and prevents rotation of the support element relative to the channel of the fixation device. The housing of the fixation device can include a closure device which can engage the channel to retain the elongate support element in the channel. The closure device can be in the form of a cap which can be fitted on to the channel. The closure device can engage the channel of the housing using mechanisms such as a thread or a twist-to-lock mechanism such as a bayonet. The cap can engage the external surface of the channel or the internal surface of the channel. A screw mechanism can extend through the closure cap or other device to engage a support element in the channel, in particular to lock it against movement within the channel. Suitable closure arrangements can use a threaded nut which can engage a thread on the external wall of a channel as in the product sold by DePuy Spine Inc under the trade mark Moss Miami, or can use a set screw which can engage the thread in the internal wall of a channel as in the product sold by DePuy Spine Inc under the trade mark Expedium, and or can use a twist-in closure mechanism as in the cap sold by DePuy Spine Inc under the trade mark Monarch Typhoon.

The transverse arm can be permanently connected to the wall of the channel. For example, it can be formed as one piece with the channel housing by a technique such as casting or machining or it can be fastened permanently to the channel housing, for example by a technique such as welding. The channel housing and the transverse arm can be provided as separate parts. For example, the side wall of the channel housing can provide a socket in to which the housing end of the transverse arm can be received. The housing end of the transverse arm can be fastened into the socket by means of cooperating threads. This can provide a suitably secure connection, particularly when the transverse arm is secured against twisting about its axis as a result of the free end of the transverse end being connected to a vertebra by a wire.

The cross-sectional area of the transverse arm will often be approximately constant over at least most of its length, with the possibility that the cross-section might vary in at least one end region to facilitate connection directly or indirectly to a vertebra or to the primary housing. For example, the cross-sectional area of the support element might be at least about 10 mm², preferably at least about 20 mm², more preferably at least about 30 mm², for example about 40 mm².

The length of the transverse arm can be selected according to the requirements of a particular application. It is expected that it will seldom be required to be longer than about 50 mm. The length of the transverse arm will usually be at least about 15 mm.

The fixation device includes a second recess in the housing or at or near the housing end of the transverse arm. The fixation device can include a second wire which is a close fit in the second recess and can be used to fasten the device to a vertebra at the housing or at or towards the housing end of the transverse arm.

The first and/or second recesses for a wire can extend at any angle to a line joining the free end and housing end of the transverse arm. Preferably, the first and/or second recesses extend approximately perpendicular to a line joining the free end and housing end of the transverse arm.

The or each recess can be provided as a channel which is open along at least part of its length or as a closed cross-section bore. An open channel is preferably provided at least in part by the upper surface of the transverse arm which faces away from the surface of the vertebra when the device is in use.

When the recess is in the form of a bore, the wire can be located so that it extends through bore before the fixation device is offered up to the vertebrae. When the recess is in the form of an open channel, it can be more convenient to offer the fixation device to the vertebra before the wire is located within the channel.

Each of the first and second recesses should be shaped so that the wire which is to be received in it is a close fit. The close fit between the wire and its respective recess allows is such that the wires is a sliding fit in the recess along the length of the wire. The close fit which is provided between the wire and the recess minimises movement of the wire other than in a direction along the length of the wire.

Preferably, the wire has a rounded cross-section, and especially has a circular cross-section. A non-circular cross section might be preferred for certain applications, for example to provide a more positive engagement with bone tissue with which it is in contact, for example by forming an indentation. The wire can comprise multiple strands in the manner of a cable. Preferably, the wire is formed as a single strand. Preferably, the wire has a solid cross-section.

Preferably, the cross-section of the wire, along at least a significant part of its length, is at least about 0.3 mm², more preferably at least about 0.5 mm². Preferably the ratio of the width of the wire to the width of the recess is not more than about 0.97, more preferably not more than about 0.95. Preferably, the ratio is at least about 0.5, more preferably at least about 0.7.

When the recess is an open channel, it is preferred that the depth of the channel is not less than half of the width of the wire.

The material of the wire will be selected from materials which are commonly used in the manufacture of wires for use in surgical fixation. Important properties of the materials include biocompatibility, tensile properties, and flexibility. Suitable materials include certain stainless steels, and titanium and alloys which contain titanium. An example of a wire which is suitable for use in the present invention is that sold by DePuy Spine Inc under the trade mark Songer. It can be particularly preferred for the wire to be formed from a shape memory alloy, taking advantage of the heat initiated recovery or the enhanced elastic properties of such materials.

The housing can further include a primary connection feature which can connect the housing to the vertebra. The primary connection feature can include, for example a bone screw or a hook. Preferably, the primary connection feature (such as for example a bone screw or a hook) extends from the base of the housing which is to engage the vertebra.

The transverse arm can provide a second connection feature. The second connection feature can be provided at or near the free end of the transverse arm. The second connection feature can be a protrusion, such as a spike. The second connection feature, for example a spike, can extend from the base of the transverse arm to engage the vertebra.

It can be preferred that the transverse arm is cranked so that the angle between the axes defined by the arms at its opposite ends (when viewed from one side of the housing generally along the superior-inferior axis) is less than 180°, for example not more than about 170°, and possibly not more than about 160°. The transverse arm is preferably cranked so as to correspond to the shape of the surface of the vertebra to which the fixation device is to be secured.

The housing can be provided with more than one transverse arm. In particular, it can be preferred that the housing is provided with a transverse arm extending from each of two opposite side walls. The angle between two such transverse arms at the points where they are connected to the housing, when viewed from above (along the axis of the channel) will generally be 180°. However, the angle might be less than 180°, for example not more than about 170°, and possibly not more than about 160°.

Each of the housing, the transverse arm and the closure cap can be made from a metallic material as is generally known for components of spinal implant kits. Suitable metals include certain stainless steels, and titanium and its alloys. It can be preferred that the transverse arm at least is formed from a material which enables the arm to be cut to length to suit a particular application.

Preferably, the support element is capable of recoverable deformation towards its original undeformed configuration (from which it had been deformed) such that the angle between its ends changes through at least about 20°, more preferably at least about 25°, especially at least about 30°. Recoverable deformation is deformation that can be recovered substantially completely back to the undeformed configuration when applied stress is removed, or otherwise when allowed to recover (for example as a result of heating to allow a transformation to austenite phase). As the support element recovers to an undeformed configuration, corrective forces are applied to the deformed spinal column to correct lateral deformation or axial rotation.

The support element will preferably be a rod, especially with a solid cross-section. A rod support element can be hollow along at least part of its length. One or more of the support elements can be a plate.

The cross-sectional area of the support element will often be approximately constant over at least most of its length, with the possibility that the cross-section might vary in at least one end region to facilitate connection directly or indirectly to a vertebra at the end or to an adjacent support element. For example, the cross-sectional area of the support element might be at least about 10 mm², preferably at least about 20 mm², more preferably at least about 30 mm², for example about 40 mm².

The support element preferably has a non-circular cross-section to enable it to fit securely in the channel housing in such a way that it can transmit torque to the housing. For example, the element can have at least one flat face. Polygonal (regular or irregular) shapes can be useful, for example with at least four faces, including square or rectangular or trapezoidal (when the element has four faces when viewed in cross-section), or with six or eight or more faces. An element which has a generally rounded cross-section might have a flat face.

The support element can be formed from a shape memory alloy. The alloy can be treated so that it is implanted while in the martensite phase. The treatment of the alloy can be such that its Aₛ and A_{f} temperatures are between ambient temperature and body temperature (37°C), so that the alloy is fully austenite phase at body temperature (for example by virtue of the A_{f} temperature being about 32°C). This allows the surgeon to make use of the thermally initiated shape recovery properties of the alloy, in which the support element is implanted in the body in the martensite phase, which is stable at ambient temperature. On implantation, the element is exposed to body temperature which leads to the phase of the alloy transforming from martensite to austenite. The element will then tend towards a configuration from which it was transformed while in the martensite phase, applying corrective forces to a patient's vertebra.

A support element which is formed from a shape memory alloy can apply corrective forces by virtue of the enhanced elastic properties that are available from such materials. Shape memory alloys can exhibit enhanced elastic properties compared with materials which do not exhibit martensite-austenite transformations and it is these properties that the present invention is concerned with in particular. The nature of superelastic transformations of shape memory alloys is discussed in "Engineering Aspects of Shape Memory Alloys", T W Duerig et al, on page 370, Butterworth-Heinemann (1990). Subject matter disclosed in that document is incorporated in this specification by this reference to the document. A principal transformation of shape memory alloys involves an initial increase in strain, approximately linearly with stress. This behaviour is reversible, and corresponds to conventional elastic deformation. Subsequent increases in strain are accompanied by little or no increase in stress, over a limited range of strain to the end of the "loading plateau". The loading plateau stress is defined by the inflection point on the stress/strain graph. Subsequent increases in strain are accompanied by increases in stress. On unloading, there is a decline in stress with reducing strain to the start of the "unloading plateau" evidenced by the existence of an inflection point along which stress changes little with reducing strain. At the end of the unloading plateau, stress reduces with reducing strain. The unloading plateau stress is also defined by the inflection point on the stress/strain graph. Any residual strain after unloading to zero stress is the permanent set of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", on page 376.

A preferred way in which non-linear superelastic properties can be introduced in a shape memory alloy involves cold working the alloy by one of several deformation methods, for example, swaging, drawing, pressing, stretching or bending. The cold working step is followed by an annealing step at a temperature less than the recrystallization temperature of the alloy, for a time sufficient to cause dislocations to rearrange, combine and align themselves (so-called "recovery" processes). The resulting recovered dislocation structure should ideally be dense enough to make plastic deformation difficult, but not so dense as to prevent the martensite phase from transforming upon the application of a load, and growing in a relatively unimpeded manner.

Since many preferred superelastic alloys are thermally unstable in the temperature range in which these recovery processes occur, a second unavoidable result of this recovery heat treatment step is to age the material, that is to cause Ni-rich particles to precipitate, having the effect of enriching the matrix phase in titanium, and thus increasing the transformation temperatures (including the A_{f} temperature). Optimum superelastic properties are only realized when using shape memory alloys above the A_{f} temperature, though it should be noted that some indications of superelasticity are observed above the Aₛ temperature (typically 2 to 20°C below A_{f}). Thus a second requirement for this recovery heat treatment is that A_{f} not be increased above the temperature at which the alloy is to be used. Practically speaking this places upper limits on the time and temperature which can be used in the recovery heat treatment.

When it is desired only to rely on the superelastic properties of an alloy without any contribution from any thermally initiated shape memory effect, the alloy should by processed so that its A_{f} temperature is below temperatures to which the alloy is likely to be subjected during implantation, that is preferably below about ambient temperature. For example, the A_{f} temperature might be not more than about 20°C.

It is often the case that a device is to be used in a shape other than that which can be practically produced by cold working processes. For example, a straight wire can be conveniently produced by cold drawing, but a wire loop or other formed shape cannot be. In this case, it is customary to form the drawn, cold worked wire into the desired "heat stable" shape, to constrain the wire in that shape, and then to perform the above described recovery heat treatment to "shape set" the component. In this case the final annealing operation has two purposes: to adjust the superelastic properties of the alloy, and to properly set the shape of the article. The time and temperature of this heat treatment step are critical. If held too long at temperature, the material over-ages, causing the A_{f} temperature to rise above the application temperature. If the annealing temperature is too short, or the temperature too low, the shape will be insufficiently formed, and too much of the original dislocation structure will remain to allow free martensite movement. This "forming" treatment may introduce still further cold work into the part, but that cold work is usually small compared to that introduced into the wire by drawing. Moreover, forming operations are often not uniform, and thus forming itself is not generally a convenient way to introduce cold work.

Articles of complicated shape require extensive forming and are very difficult to produce according to the above process. If the forming process causes strains which are too severe, the article will fracture as it is heated to the shape setting and recovery temperature (one is able to restrain the formed article, but cannot maintain its shape during the heating process without causing fracture). It is possible to overcome this problem by performing a series of smaller, intermediate shape setting operations which accumulate to provide the desired final shape, but unfortunately each of these shape setting operations requires sufficient annealing time to allow the material to soften, in preparation for the next. When accumulated, these heat treatments cause a cumulative ageing effect that can cause the A_{f} temperature to rise beyond the expected service temperature (37°C for most medical applications, for example).

It is also known that one can introduce superelasticity by solution treating and ageing, abandoning all attempts to retain cold work. Although this approach resolves the above problems, it leads to inferior superelastic properties, producing articles that are susceptible to fatigue and storage problems.

Examples of shape memory alloys which might be used in the first and possibly other support elements in the kit of the invention include nickel-titanium based alloys, especially the binary alloy which contains 50.8 at-% nickel. Suitable alloys include those which satisfy ASTM F2063-00. It will often be particularly preferred for both the first and second support elements to be formed from shape memory alloys, especially for each support element to be formed from shape memory alloys. Other metals which might be used to form support elements which do not exhibit shape memory properties include titanium and alloys thereof, for example Ti6Al4V alloys such as satisfy ASTM F136-02a or ASTM F 1472-02a or both.

Materials which exhibit shape memory properties, other than alloys, can be used. For example, polymeric materials can be used. Shape memory properties can be imparted to polymeric materials by forming them in a desired ultimate shape (for example by moulding), crosslinking the material, heating the material to a temperature at which it softens, deforming the material while soft and restraining the material in the deformed configuration while it cools. The material will tend to revert towards the initial "as formed" configuration when reheated. Examples of suitable polymeric materials which can be used in this way include oligomers, homopolymers, copolymers and polymer blends which include, as monomers, 1-, d- or d/1-lactide (lactic acid), glycolide (glycolic acid), ethers, ethylene, propylene and other olefins, styrene, norbornene, butadiene, polyfunctional monomers such as acrylates, methacrylates, methyl acrylates, and esters such as caprolactone. The use of such polymeric materials in related applications is disclosed in WO-02/34310.

Embodiments of the invention will now be described by way of example with reference to the accompanying drawings, in which:
Figure 1 is an isometric view of a fixation device of the invention.
Figure 2 is an isometric view of the fixation device of figure 1 attached to a vertebra.
Figure 3 is an isometric view of the fixation device of figure 1 without the closure cap.
Figure 4 is a view from above of the kit of the invention including the fixation device of figure 1.

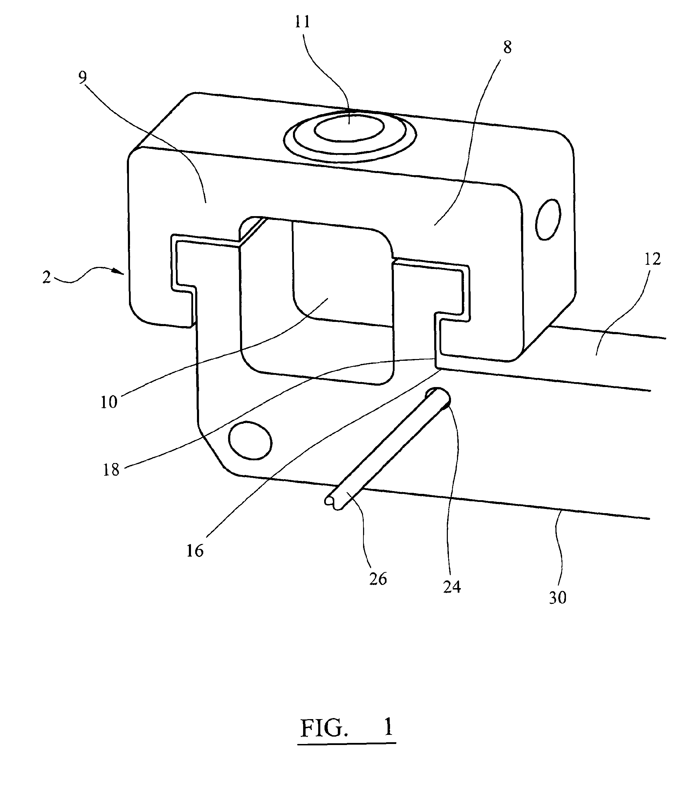

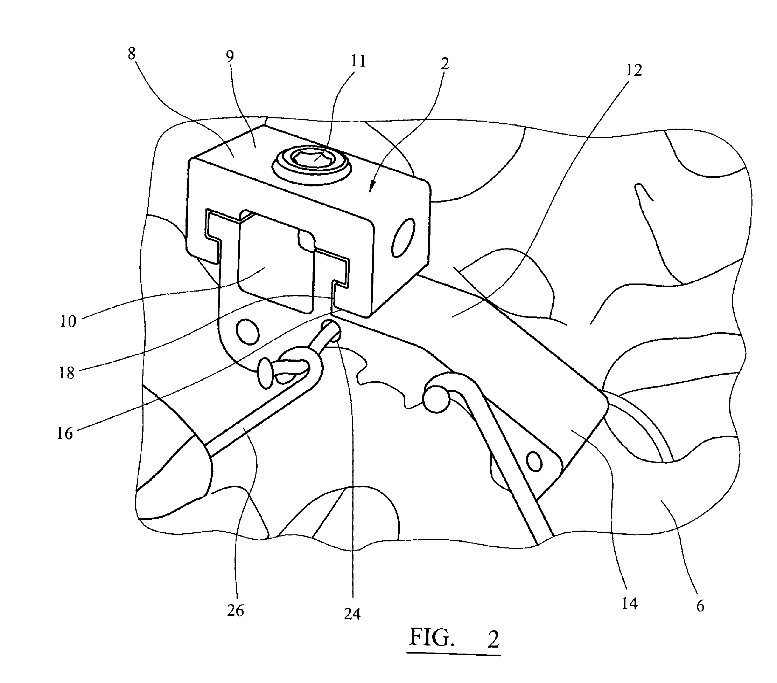

Referring to the drawings, Figures 1 to 4 show a fixation device 2 which can be used to secure an elongate spinal support rod 4 to a patient's vertebra 6. The device 2 comprises a housing 8 for the support rod 4. The housing 8 is formed from stainless steel of a type which is commonly used in the manufacture of implant components. The fixation device 2 includes a closure cap 9 which slidingly engages the upper portion of the housing 8. The housing 8 and closure cap 9 together form a channel 10 for receiving the rod 4. The channel 10 has a generally rectangular cross-section when viewed along a line joining the ends of the channel 10. The upper surface of the closure cap 9 defines a threaded bore (not shown) to threadingly engage a threaded portion of a screw 11.

The housing 8 is permanently attached to a transverse arm 12. The transverse arm 12 is formed from a stainless steel. The transverse arm 12 has a free end 14 and a housing end 16. The housing end 16 of the transverse arm is attached to a side wall 18 of the channel housing 8. The transverse arm 12 extends from the side wall 18 of the housing 8 in a direction which is substantially perpendicular to a line joining the ends 14 and 16 of the channel 10. The transverse arm 12 is cranked so that the base of the transverse arm 12 is shaped to conform to the upper surface of the vertebra 6 to which the fixation device 2 is to be attached. The cross-sectional area of the transverse arm 12 is approximately constant over most of its length.

The free end 14 of the transverse arm 12 provides a first recess 20. First recess 20 is an open channel. First recess 20 is provided by the upper surface of the transverse arm 12. First recess 20 extends across the upper surface of the transverse arm 12 in a direction which is substantially perpendicular to a line joining the free end 14 and housing end 16 of the transverse arm 12. First recess 20 is dimensioned and shaped to receive a first wire 22.

The fixation device 2 includes a second recess 24 at the housing end 16 of the transverse arm 12. The second recess 24 is a bore and extends substantially perpendicular to a line joining the ends 14 and 16 of the transverse arm 12. The bore 24 is dimensioned and shaped to receive a second wire 26.

The free end 14 of the transverse arm 12 has a spike 28 extending from the base 30 of the arm 12. The spike 28 extends substantially perpendicular to the base 30 of the transverse arm 12.

The implant kit includes a support rod 4 having a rectangular cross-section when viewed along a line joining the ends of the rod 4. The cross-section of the support rod 4 is dimensioned to be received within the channel 10 of housing 8 of the fixation device.

The support rod 4 has a solid cross-section and the cross-sectional area of the rod 4 is approximately constant over most of its length. The support rod 4 is composed of a shape memory alloy. The support rod 4 can be twisted and bent into a deformed configuration. The support rod 4 is capable of recoverable deformation towards its undeformed configuration.

In use, a surgeon aligns the support rod 4 along the length of the deformed spinal column. The support rod 4 is twisted and bent into a deformed configuration which corresponds to the shape of the deformed spinal column. The support rod 4 is positioned at the desired location against the spinal column. Prior to securing a fixation device 2 to the spinal column, the surgeon inserts second wire 26 into bore 24. The second wire 26 is a close fit in the bore 24. The surgeon is able to move the second wire 26 within the bore 24 in a direction parallel to the longitudinal axis of the second wire 26. The bore 24 restricts the movement of the second wire 26 within the bore 24 within the plane of the adjacent portion of the transverse arm 12 in a direction which forms an angle with the longitudinal axis of the wire 26. The bore 24 also restricts the movement of the second wire 26 within the bore 24 in a direction which forms an angle with the plane of the adjacent portion of the transverse arm 12. After insertion of the second wire 26, the surgeon inserts the support rod 4 into channel 10 of the housing 8 of the fixation device 2. The closure cap 9 slidingly engages the upper portion of the housing 8. The surgeon adjusts the position of the fixation device 2 to the desired location relative to a vertebra 6 by sliding the housing 8 relative to the support rod 4.

When the surgeon has positioned the fixation device 2 in the desired location a screw 11 is inserted through the threaded bore (not shown) of the closure cap 9. The screw 11 is tightened until the screw 11 engages the support rod 4. The support rod 4 is fixed in position relative to the vertebra 6 by the screw 11. The rectangular cross-section of the support rod 4 prevents the support rod 4 from being able to rotate within the channel 10 of the housing 8.

The second wire 26 is then looped around an end of the vertebra 6. The surgeon tightens the second wire 26 so that the base of the housing 8 is connected directly to the vertebra 6. The first wire 22 is then inserted through open channel 20 at the free end 14 of the transverse arm 12. The first wire 22 is a close fit in the open channel 20. The surgeon is able to move the first wire 22 within the open channel 20 in a direction parallel to the longitudinal axis of the first wire 22. The open channel 20 restricts the movement of the first wire 22 within the open channel 20 within the plane of the adjacent portion of the transverse arm 12 in a direction which forms an angle with the longitudinal axis of the first wire 22. The first wire 22 is looped around the opposite end of the vertebra 6 so that the transverse arm 12 is connected directly to the vertebra 6. The spike 28 at the free end 14 of the transverse arm 12 engages and penetrates the vertebra 6. The base 30 of the transverse arm 12 contacts the upper surface of the vertebra 6. The surgeon then attaches one or more additional fixation devices to the support rod 4 to secure the support rod 4 to adjacent vertebrae 6.

The first and second wires 22 and 26 secure the fixation device 2 in position relative to the vertebra 6 so that the support rod 4 is prevented from rotating relative to the spinal column. Once the body has been secured to the vertebra 6, the temperature of the support rod 4 increases. The alloy which forms the support rod 4 transforms from martensite to austenite. This phase transformation of the alloy causes the support rod 4 to transform from its deformed configuration to its original undeformed configuration. As the support rod 4 begins to change configuration, for example by untwisting and straightening, the spinal column is subjected to corrective forces. As the support rod 4 transforms to its original undeformed configuration the adjacent vertebrae of the spinal column will be subjected to corrective forces which cause the deformed spinal column of the patient to straighten to its correct position.

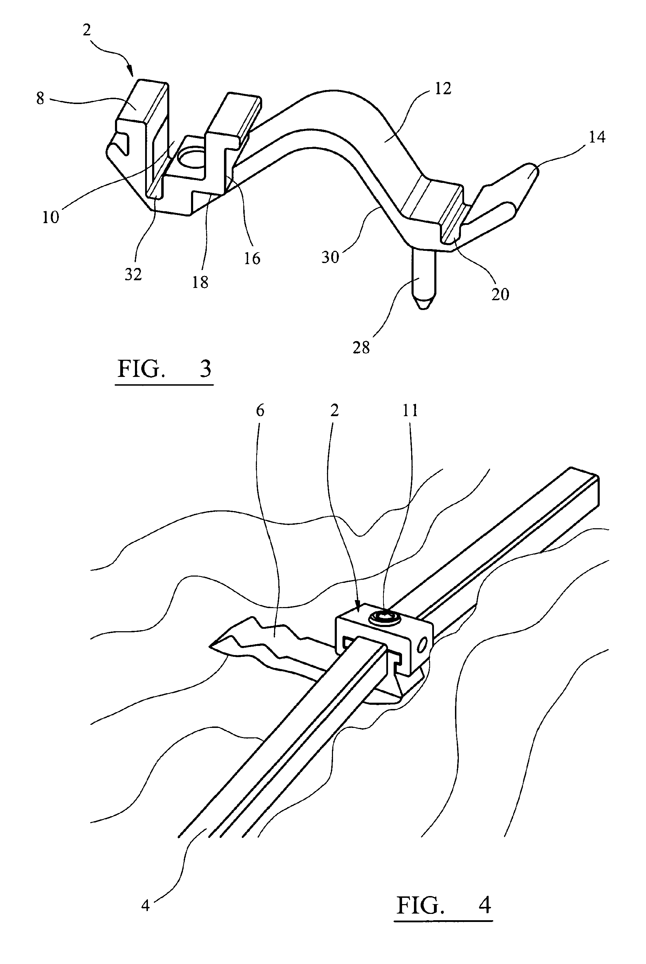

As shown in Figure 3, the transverse arm can be arched when viewed from one side (along the superior-inferior axis) to allow it to be placed over the spinous process. In the embodiment shown in Figure 3, the second recess 32 for a second wire is provided in the base of the housing in which the support element can be received.

## Claims

1. A fixation device for connecting an elongate support element to a patient's vertebra in order to correct a spinal deformity, which comprises:
a. a housing for the support element formed by a channel in which the element can be received,
b. a transverse arm having a housing end and a free end, which is connected to a side wall of the channel housing at its housing end, in which the transverse arm has a first recess formed in it towards its free end, and in which the housing or the transverse arm at or towards the housing end has a second recess formed in it,
c. a first wire which is a close fit in the first recess and can engage a vertebra so that the transverse arm is connected directly to the vertebra towards its free end,
d. a second wire which is a close fit in the second recess and can engage a vertebra so that the device is connected directly to the vertebra at the housing at or towards the housing end of the transverse arm.

2. A device as claimed in claim 1, in which the housing includes a closure device which can engage the channel to retain the elongate support element in the channel.

3. A device as claimed in claim 1, in which the channel formed by the housing has at least one flat internal face.

4. A device as claimed in claim 1, in which at least one of the recesses is a bore or an open channel.

5. A device as claimed in claim 1, further including a primary connection feature extending beyond the base of the housing to engage the vertebra directly.

6. A device as claimed in claim 1, further including a second connection feature extending beyond the base of the transverse arm to engage the vertebra directly.

7. A device as claimed in claim 6, in which the second connection feature is a spike.

8. A device as claimed in claim 1, in which the transverse arm is cranked.

9. A device as claimed in claim 1, in which the transverse arm is permanently connected to the wall of the channel.

10. A device as claimed in claim 1, in which the transverse arm is arched when viewed from one side along the superior-inferior axis to allow it to be placed over the spinous process.

11. An implant kit including a fixation device as claimed in claim 1 and an elongate support element.

12. A kit as claimed in claim 11, in which the elongate support element is composed of a shape memory alloy.

13. A kit as claimed in claim 11, in which the support element has a non-circular cross-section.
